# FASCICULE DE BREVET EUROPEEN

(11) **EP 3 242 710 B1**
(45) Date de publication et mention de la délivrance du brevet: **12.06.2019**
(21) Numéro de dépôt: 16700178.3
(22) Date de dépôt: 07.01.2016
(51) Int. Cl.: A61N 1/04, A61N 1/30, A61N 1/20, A61N 1/32, H01R 13/15, H01R 13/24

(54) **DISPOSITIF POUR L'ÉLECTROSTIMULATION ET/OU L'IONTOPHORÈSE COMPRENANT UN SUPPORT ET UN BOITIER**
VORRICHTUNG ZUR ELEKTROSTIMULATION UND/ODER IONTOPHORESE MIT EINEM TRÄGER UND EINER BOX
DEVICE FOR ELECTROSTIMULATION AND/OR IONTOPHORESIS COMPRISING A SUPPORT AND A BOX

(30) Priorité: 07.01.2015 FR 1550116
(43) Date de publication de la demande: 15.11.2017
(73) Titulaire: Feeligreen, 06130 Grasse (FR)
(72) Inventeur: BIANCHI, Christophe, 06100 Nice (FR)
(74) Mandataire: Brizio Delaporte, Allison
(86) Numéro de dépôt international: PCT/EP2016/050231
(87) Numéro de publication internationale: WO 2016/110550

(56) Documents cités:
- EP-A1- 2 319 577
- FR-A1- 2 778 108
- US-A- 5 645 526
- US-A- 6 157 858
- US-A1- 2006 247 730
- US-A1- 2013 281 914
- US-A1- 2014 039 378

## Description

### DOMAINE DE L'INVENTION

L'invention concerne un dispositif pour l'électrostimulation et/ou l'iontophorèse comprenant un support et un boitier.

L'invention concerne les techniques consistant à soumettre des cellules dermiques à un champ électrique pour augmenter leur activité et/ou à utiliser un courant électrique pour faciliter la diffusion transdermique de substances actives, et concerne plus particulièrement un dispositif d'électrostimulation et/ou iontophorèse.

### ETAT DE LA TECHNIQUE

La stimulation cellulaire par un champ électrique ou électrostimulation est utilisée dans de nombreuses applications. Elle est connue pour diminuer la douleur ou bien pour des applications cosmétiques comme le traitement des rides. On soumet par exemple des fibroblastes à un champ électrique pour augmenter leur taux de production de protéine. Les protéines produites par les fibroblastes étant essentiellement le collagène et l'élastine, l'effet attendu est une amélioration de l'élasticité de la peau et donc un effet visible de réduction des rides. L'électrostimulation peut aussi être utilisée pour le traitement de la cicatrisation pour laquelle le champ électrique augmente la migration cellulaire et facilite ainsi la cicatrisation.

L'iontophorèse quant à elle est une technique qui peut être utilisée à des fins cosmétiques et/ou médicales pour introduire des substances actives dans le derme de la peau au moyen d'un courant.

Contrairement aux modes d'administration traditionnels par voie orale ou parentérale, la durée d'application du courant pour l'iontophorèse ou l'électrostimulation est longue. La source de courant doit donc être adaptée pour fournir un courant pendant une longue période. En outre, ces dispositifs nécessitent également des moyens de contrôle du courant fourni, par exemple sous forme d'un dispositif électronique pouvant être programmé en fonction du traitement ou de l'effet recherché. Le dispositif électronique et la source de courant sont associés à l'élément destiné à être en contact avec la peau. Ces dispositifs sont donc souvent imposants par leur taille avec une source de courant et un dispositif électronique encombrants.

Ces dispositifs sont chers et sont ainsi réutilisés pour plusieurs patients, les éléments étant en contact avec la peau devant alors être nettoyés voir stérilisés pour assurer les règles de sécurité sanitaire. Par ailleurs, dans le cadre d'une utilisation iontophorètique, il est souvent difficile de modifier l'actif à administrer. On se retrouve alors avec un dispositif complet pour chaque actif à administrer.

US 2013/0281914 et US 2006/0247730 montrent un dispositif selon le préambule de la revendication 1.

Il existe donc le besoin de proposer un dispositif qui permette une utilisation individuelle voire à usage unique tout en limitant les coûts.

### RESUME DE L'INVENTION

A cet effet la présente invention concerne un dispositif d'électrostimulation ou d'iontophorèse selon la revendication 1. Les revendications dépendantes 2-14 définissent des modes de réalisation préférés de l'invention. L'invention concerne également un procédé d'assemblage d'un dispositif tel que défini par la revendication 15.

Les autres aspects, modes, possibilités préférées ou exemples divulgués dans la présente description écrits qui ne tombent pas sous la définition des revendications ne font pas partie de la présente invention.

Le dispositif comprend un boitier et un support amovible comprenant une première face destinée à être en regard de la peau de l'utilisateur et une deuxième face opposée à la première et destinée à être connectée électriquement au boitier. La première face du support comprend des électrodes et avantageusement un circuit imprimé reliant les électrodes. Selon l'invention, le boitier comprend des moyens de connexions électriques, lesdits moyens de connexion électriques sont au moins un connecteur électrique, optionnellement à ressort, agencé sur une première face du boitier et destiné à assurer la connexion électrique avec le support.

Ce dispositif permet d'assurer une connexion électrique de bonne qualité entre le boitier et le support et ceci même lorsque l'assemblage est assuré par un utilisateur peu expérimenté ou peu habile. Les coûts de mises en oeuvre sont faibles et l'encombrement est adapté aux faibles dimensions du dispositif et plus spécifiquement du boitier.

De plus ces connecteurs à ressort ont montré des bonnes tenues à l'oxydation.

De manière surprenante, ces connecteurs à ressort sont particulièrement adaptés à un support souple qui vient se conformer sur le boitier rigide et lesdits connecteurs à ressort.

Avantageusement, le boitier comprend une encoche comprenant les connecteurs à ressorts et dans laquelle se place au moins partiellement le support.

Selon une possibilité préférée, le dispositif comprend une recharge assurant le maintien du support dans l'encoche du boitier.

Selon un autre exemple, la présente description concerne un dispositif d'électrostimulation ou d'iontophorèse comprenant un boitier et un support amovible comprenant une première face destinée à être en regard de la peau de l'utilisateur et une deuxième face opposée à la première et destinée à être connectée électriquement au boitier. La première face du support comprend des électrodes et avantageusement un circuit imprimé reliant les électrodes. Le boitier comprend des moyens de connexions électriques, lesdits moyens de connexion électriques sont au moins un connecteur électrique, optionnellement à ressort, agencé sur une première face du boitier et destiné à assurer la connexion électrique avec le support.

Selon un mode de réalisation avantageux, le dispositif comprend une recharge amovible configurée pour coopérer mécaniquement avec le boitier de sorte à maintenir le support entre le boîtier et la recharge et de sorte à maintenir le support en connexion avec le au moins un connecteur électrique.

Ainsi le support est enserré entre le boîtier et la recharge. Lorsque la recharge amovible est fixée sur le boîtier elle retient mécaniquement le support.

De préférence la coopération mécanique entre la recharge amovible et le boitier s'effectue par emboitement de l'un parmi la recharge et le boitier dans une forme complémentaire telle qu'une encoche portée par l'autre parmi la recharge et le boitier. La forme complémentaire telle qu'une encoche peut ainsi être portée par la recharge ou par le boitier.

La recharge comprend au moins un élément actif cosmétique ou thérapeutique destiné à diffuser sur le support.

### BREVES DESCRIPTION DES FIGURES

Les dessins sont donnés à titre d'exemples et ne sont pas limitatifs de l'invention. Ils constituent des représentations schématiques de principe destinées à faciliter la compréhension de l'invention et ne sont pas nécessairement à l'échelle des applications pratiques.

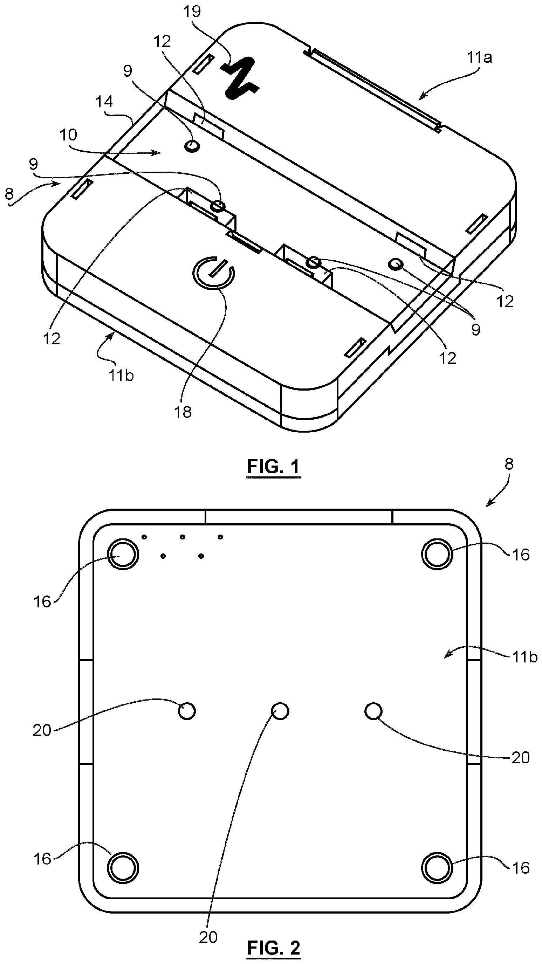

La figure 1 représente le boitier du dispositif selon l'invention vue de la première face.
La figure 2 représente le boitier du dispositif selon l'invention vue de la deuxième face.

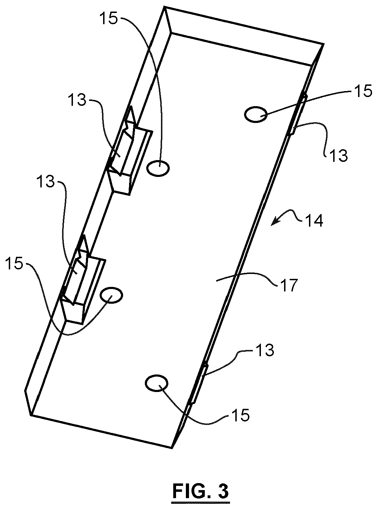

La figure 3 représente la recharge du dispositif selon l'invention vue du dessous.
La figure 4 représente une vue de dessus d'un mode de réalisation du support étant transparent et ayant une forme carré connecté au dispositif.
La figure 5 représente une vue d'un autre mode de réalisation du support, ledit support étant transparent et ayant la forme de deux carrés connectés par des pattes de connexion au boitier. Dans cette réalisation, la recharge amovible n'est pas fixée sur le boitier.
La figure 6 représente un autre mode de réalisation selon la figure 5 avec un support plus grand.
Les figures 7 et 8 représentent des connecteurs à ressort selon deux modes de réalisation.

### DESCRIPTION DETAILLEE DE L'INVENTION

Avant d'entamer une revue détaillée de modes de réalisation, sont énoncées ci-après des caractéristiques optionnelles qui peuvent éventuellement être utilisées en association ou alternativement.

Avantageusement, le support comprend une nappe souple.

Avantageusement, la nappe souple présente une surface supérieure à la surface du boitier.

Avantageusement, le connecteur à ressort est un plot de connexion à ressort.

Avantageusement, le boitier comprend une pluralité de connecteur à ressort.

Avantageusement, le support comprend sur sa première face un circuit électrique reliant les électrodes et en ce que le support comprend sur sa première face, un élément conducteur destiné à coopérer avec le au moins un connecteur à ressort.

Avantageusement, l'élément conducteur est une portion du circuit électrique.

Avantageusement, comprenant un film de matériau isolant électriquement destiné à isoler électriquement ledit circuit électrique de la peau sur laquelle le dispositif est destiné à être appliqué.

Avantageusement, le circuit électrique et les électrodes sont formés par une encre conductrice imprimée ou sérigraphiée sur la première face du support.

Avantageusement, le boitier comprend sur sa première face une encoche destinée à recevoir au moins partiellement le support et dans laquelle est agencé le au moins un connecteur à ressort.

Avantageusement, le dispositif comprend une recharge amovible configurée pour coopérer avec l'encoche du boitier de sorte à maintenir le support au contact du au moins un connecteur à ressort du boitier.

Avantageusement, le dispositif comprend une recharge amovible configurée pour coopérer avec l'encoche du boitier de sorte à maintenir le support entre le boîtier et la recharge. Ainsi le support est enserré entre le boîtier et la recharge. Lorsque la recharge amovible est fixée sur le boîtier elle retient mécaniquement le support.

Selon un mode de réalisation alternatif, le dispositif comprend une recharge amovible configurée pour coopérer mécaniquement avec le boitier de sorte à maintenir le support entre le boîtier et la recharge. Ainsi le support est enserré entre le boîtier et la recharge. Lorsque la recharge amovible est fixée sur le boîtier elle retient mécaniquement le support.

De préférence la coopération mécanique entre la recharge amovible et le boitier s'effectue par emboitement de l'un parmi la recharge et le boitier dans une encoche portée par l'autre parmi la recharge et le boitier. L'encoche peut ainsi être portée par la recharge ou par le boitier.

Avantageusement, la recharge comprend au moins un élément actif cosmétique ou thérapeutique destiné à diffuser sur le support.

Avantageusement, le boitier comprend sur une deuxième face opposée à la première face des connecteurs électriques fixes destinés à être en connexion électrique avec le support.

Avantageusement, le support comprend au moins un élément actif cosmétique ou thérapeutique disposé sur sa première face.

Avantageusement, le support comprend une trame en biocellulose imbibé sur la première face du support d'au moins un élément actif.

Avantageusement, le support comprend une trame textile étant recouverte sur la première face du support d'un hydrogel contenant au moins un élément actif.

Avantageusement, le support comprend un adhésif sur sa première face de sorte à maintenir le dispositif appliqué sur la peau.

La présente description concerne également une recharge pour dispositif pour l'électrostimulation et/ou l'iontophorèse tel que décrit ci-dessus caractérisée en ce que la recharge est rigide et vient se fixer par clip dans l'encoche du boitier de sorte à maintenir le support en connexion avec le au moins un connecteur à ressort.

La présente description concerne également un ensemble comprenant un dispositif selon l'invention et au moins une recharge configurée pour se fixer par clip au boitier, par exemple pour se fixer par clip dans une encoche formée par le boitier de sorte à maintenir le support en connexion avec le au moins un connecteur à ressort.

Avantageusement, la recharge comprend un fond destiné à être en contact du support comprenant préférentiellement des reliefs assurant le contact entre le au moins un connecteur à ressort du boitier et le support.

La présente description concerne un dispositif configuré pour l'électrostimulation et/ou l'iontophorèse. Le dispositif comprend un support 1 comprenant une première face 2a et une deuxième face 2b opposée. Préférentiellement, le dispositif comprend un boitier 8. Avantageusement, le boitier 8 comprend un module électronique 3. Le boitier 8 comprend une première face 11a et une deuxième face 11b. Le boitier 8 et le support 1 sont destinés à être en contact et avantageusement connectés électriquement préférentiellement par la première face 11a du boitier 8. Selon une possibilité, le boitier 8 est destiné à être appliqué sur l'utilisateur par sa deuxième face 11b. La première face 11a comprend avantageusement des moyens d'activation tel que par exemple un bouton de commande central 18 permettant l'allumage, l'extinction et éventuellement le contrôle du dispositif. Un signal lumineux 19 d'indication du bon fonctionnement du dispositif peut être prévu sur la première face 11a, avantageusement destinée à être en visible par l'utilisateur une fois le dispositif appliqué sur la peau, par exemple une diode électroluminescente.

Le support 1 est amovible. Le support 1 peut être en contact avec le boitier 8 puis séparé aisément.

Le support 1 est destiné à être appliqué sur la peau de l'utilisateur par sa première face 2a. Le support 1 comprend des électrodes 4 sur la première face 2a de sorte à être au contact de la peau. Ces électrodes 4 permettent de transmettre un courant électrique à la peau. Les électrodes 4 sont avantageusement affleurant à la surface de la première face 2a. Selon une autre possibilité, les électrodes sont en reliefs ou en creux au niveau de la première face 2a.

Le support 1 comprend un circuit électrique imprimé 6 destiné à connecter les électrodes 4. Avantageusement, le circuit imprimé 6 est disposé sur la première face 2a du support 1. Préférentiellement, le circuit imprimé 6 est isolé électriquement de la peau sur laquelle le dispositif est destiné à être appliqué. A titre d'exemple une couche de matériau isolant est appliquée sur le circuit imprimé 6. La couche de matériau isolant exclu les électrodes 4.

On entend par « en contact », un contact direct ou indirect. Dans le cas d'iontophorèse, la première face 2a du support 1 n'est pas directement appliquée sur la peau, le contact est indirect, par exemple par l'intermédiaire d'une couche contenant des éléments actifs. Des exemples de couches et d'éléments actifs sont donnés plus avant dans la description. Si le dispositif est utilisé comme dispositif d'électrostimulation, la première face 2a du support 1 est appliquée directement sur la peau de l'utilisateur sans couche intermédiaire contenant des éléments actifs.

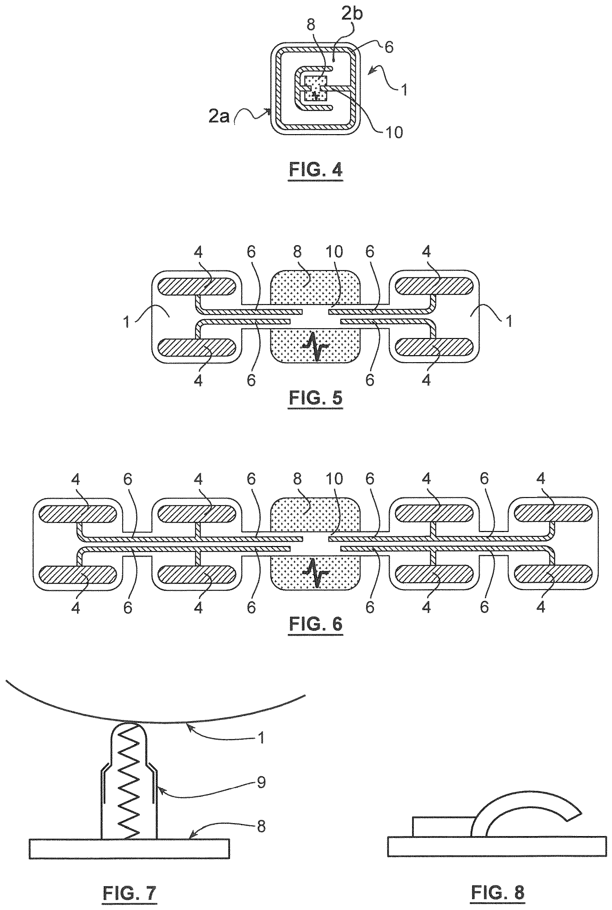

Le boitier 8 selon l'invention comprend des moyens de connexions électriques comprenant au moins un, préférentiellement plusieurs connecteurs à ressort 9. Ces connecteurs à ressort 9 sont destinés à assurer la connexion électrique avec le support 1. Les connecteurs à ressort 9 peuvent être de différentes formes telles qu'illustrées aux figures 7 et 8. Les connecteurs à ressort 9 sont par exemple des plots tels qu'illustrés à la figure 7.

Selon un mode de réalisation préféré, le boitier 8 comprend une encoche 10 formée sur sa première face 11a. Cette encoche 10 est destinée à recevoir au moins partiellement le support 1. Préférentiellement, les connecteurs à ressort 9 sont agencés dans l'encoche 10. Telle qu'illustrée sur les figures, l'encoche 10 est une partie évidée du boitier 8 sur la première face 11a. L'encoche 10 est préférentiellement de forme rectangulaire. L'encoche 10 s'étend avantageusement sur toute la largeur du boitier 8. L'encoche 10 permet la réception du support 1, avantageusement le support 1 est de dimensions supérieures aux dimensions du boitier 8 de sorte qu'une première portion seulement du support 1 soit placée dans l'encoche 10 et qu'une deuxième portion, le reste, du support 1 dépasse hors du boitier 8. La deuxième portion du support est destinée à être en contact avec la peau. Ses dimensions sont suffisantes pour dépasser du boitier 8 et venir en contact avec la peau de l'utilisateur.

La surface totale du support 1 est supérieure à la surface du boitier 8 c'est-à-dire que la surface de la deuxième face 11b du boitier 8 est supérieure à surface de la première face 2a du support 1.

Le support 1 s'applique dans l'encoche 10 par sa première face 2a. Avantageusement, le support 1 comprend un élément conducteur destiné à relier le boitier 8 et le support 1, plus précisément le module électronique 3 au circuit imprimé 6. Plus précisément, la première face 2a comprend au moins en élément conducteur destiné à être en contact d'au moins un connecteur à ressort 9.

Le circuit imprimé 6, les électrodes 4 et l'élément conducteur sont formés sur la premier face 2a du support 1. Ceci permet une fabrication aisée avec des dépôts de couches électroniques sur une seule face du support 1.

A titre d'exemple les électrodes 4, le circuit imprimé 6 et avantageusement l'élément conducteur sont formés par impression avec une encre conductrice sur le support 1. A titre d'exemple des encres conductrices à bases d'argent, d'or, de carbone ou d'oxyde de zinc peuvent être utilisées. Préférentiellement, les électrodes 4 destinées à être en contact avec l'utilisateur sont en matériau bioconducteur tel que par exemple le PEDOT-PSS (poly(3,4-éthylènedioxythiophène) - poly(styrène sulfonate) de sodium. Le circuit électrique imprimé 6 peut également être en fibres conductrices tissés dans un textile.

L'application du support 1 dans l'encoche 10 entraine la mise en contact de l'élément conducteur du support 1 et du connecteur à ressort 9. L'élément conducteur est classiquement une portion du circuit électrique 6 éventuellement exclue de la couche de matériau isolant recouvrant le circuit électrique 6. Selon une possibilité, le poids du support 1 entraine l'écrasement du ressort du connecteur à ressort 9 initiant la conduction électrique entre boitier 8 et le support 1, plus précisément entre l'élément conducteur du support 1 et le connecteur à ressort 9 du boitier 8.

Selon une autre possibilité, le dispositif comprend un élément de mise en contact du support 1 sur au moins un connecteur à ressort 9. Par exemple, cet élément de mise en contact est une recharge 14. La recharge 14 est configurée pour coopérer avec l'encoche 10 du boitier 8. La recharge 14 est amovible. Préférentiellement, elle est fixée au boitier 8 par des fixations rapides par exemple des clips. La recharge 14 se place dans l'encoche 10 de sorte à maintenir en position le support 1 dans l'encoche 10. La recharge 14 en se fixant dans l'encoche 10 exerce un appui sur le support 1 et plus précisément sur sa première portion placée dans l'encoche 10 assurant l'appui du support 1 sur les connecteurs à ressort 9. A titre préféré, la recharge 14 comprend sur son fond 17 des reliefs 15. Les reliefs 15 sont préférentiellement proéminents de sorte à assurer un appui plus important sur les connecteurs à ressort 9.

La recharge 14 est avantageusement de forme complémentaire à l'encoche 10. La recharge 14 est un élément rigide. Selon un mode de réalisation, la recharge 14 comprend un au moins un élément actif cosmétique ou thérapeutique. L'élément actif est destiné à diffuser sur le support 1 de sorte à être administrer à l'utilisateur. La recharge 14 est un réservoir dont le fond 17 destiné à être en contact avec le support 1 est configuré pour permettre la diffusion de l'élément actif, par exemple par la présence d'ouverture ou de matériau poreux. Avant usage, la recharge 14 et plus spécifiquement le fond 17 de la recharge 14, est fermé, de sorte à protéger l'élément actif et obturer le réservoir. Selon une possibilité, la recharge 14 est vide. Il est possible de placer un élément actif directement au niveau du support 1.

Le boitier 8 comprend selon un mode de réalisation additionnel ou alternatif, des connecteurs électriques fixes 16. Ces derniers sont agencés sur la deuxième face 11b du boitier 8. Ces connecteurs électriques fixes 16, par exemple des pointes en inox, peuvent être avantageusement au moins quatre et disposés avantageusement en carré. Ces connecteurs électriques fixes 16 sont avantageusement destinés à délivrer directement le courant à l'utilisateur, sans passer par un support. Selon une autre possibilité, ces connecteurs électriques fixes 16 sont destinés à coopérer et à connecter le boitier 8 à un support 1 agencé sur la deuxième face du boitier 8

Le dispositif selon l'invention est maintenu en contact avec la peau par des moyens d'attache. Les moyens de fixation sont par exemple des rubans ou fils pouvant être élastiques et enserrant la zone d'application. A titre préféré, les moyens d'attache comprennent un matériau adhésif appliqué sur la première face 2a du support 1 de sorte à maintenir le support 1 sur la peau par l'adhésif. Préférentiellement, l'adhésif est un matériau adhésif sur ces deux faces pour coopérer avec la première face 2a du support et avec la zone de la peau sur laquelle le support doit être appliqué. L'adhésif est avantageusement biocompatible, par exemple un polymère adhésif à base aqueuse. Préférentiellement, l'adhésif est formulé pour contenir l'actif cosmétique ou thérapeutique à appliquer.

Selon un mode de réalisation avantageux, le module électronique 3 comprend des moyens de mesures de paramètres physiologiques de l'utilisateur. Préférentiellement, au moins un capteur 20, par exemple de mesure de la température, est agencée sur le boitier 8. Préférentiellement, le capteur 20 est placé sur la deuxième face 11b du boitier 8 opposée à la première face 11a. De cette manière, le capteur 20 est avantageusement agencé au contact de la peau une fois le dispositif appliqué sur l'utilisateur. Les capteurs 20 peuvent mesurer également le pH, la pression, l'humidité relative, la résistivité...

Le module électronique 3 est avantageusement destiné à contrôler le courant fourni. Le module électronique 3 permet avantageusement de contrôler l'intensité, la tension et/ou la durée d'administration. Selon une possibilité, le module électronique 3 comprend un microprocesseur alimenté par une source d'énergie électrique telle qu'un accumulateur rechargeable ou une pile et qui commande l'application d'une tension aux électrodes 4 par exemple par l'intermédiaire d'un générateur de tension.

La source d'énergie peut être diverse. Avantageusement, la source d'énergie est autonome, à titre d'exemple un générateur autonome d'énergie par récupération d'énergie peut être utilisé ou bien des batteries de type piles.

Selon un mode de réalisation avantageux, le module électronique 3 comprend la source de courant, préférentiellement une source de courant autonome. Cette disposition est particulièrement avantageuse pour permettre un usage ambulatoire du dispositif selon l'invention.

Le dispositif de l'invention comprend un élévateur de tension. L'élévateur de tension est avantageusement présent dans le module électronique.

L'élévateur de tension permet d'élever la tension fournie aux électrodes à partir de la tension délivrée par la source d'énergie. Classiquement, les sources d'énergie utilisées pouvant être autonome et/ou portable sont de faible encombrement et fournissent des tensions comprises entre 1 et 3 V. Or, dans certaines cas, pour l'électrostimulation ou l'iontophorèse, la tension nécessaire à l'obtention d'une intensité de 100 à 800µA peut aller jusqu'à 30V.

Le dispositif selon l'invention est capable de créer une intensité de courant avantageusement 10 fois supérieure à l'intensité issue de la source d'énergie.

Préférentiellement, le dispositif comprend des moyens de sécurité destinés à assurer la sécurisation du dispositif dans tous les modes de fonctionnement prévus ou non. Notamment, le courant ne doit pas avoir une intensité supérieure à 1mA dans une quelconque des électrodes et ceci en fonctionnement normal avec une résistivité du derme variable ou en cas d'anomalie d'une ou plusieurs électrodes ou en cas de circuit ouvert du fait du décollement d'une électrode ou en cas de mise en coupe -circuit d'une ou de plusieurs électrodes, en cas de chute brutale de la résistivité du derme ou en cas de défaillance du dispositif.

A cet effet, le dispositif comprend préférentiellement, des moyens mécaniques redondants destinés à limiter les risques dus à la défaillance d'un élément mécanique. Préférentiellement, le dispositif comprend des moyens logiciels destinés à assurer un contrôle du module électronique 3.

A cet effet, le dispositif comprend préférentiellement, des moyens électroniques redondants destinés à limiter les risques dus à la défaillance d'un élément électronique ou du logiciel de contrôle embarqué dans le microprocesseur. Préférentiellement, le dispositif comprend des moyens logiciels destinés à assurer un contrôle du module électronique 3. Les moyens logiciels comprennent un logiciel embarqué dans une unité de calcul tel que le microprocesseur. Ce logiciel assure une mesure permanente de paramètres clefs de fonctionnement et de sécurité tels que par exemple la densité de courant aux électrodes, la résistivité du derme, la température. Ce logiciel pilote l'élévateur de tension afin d'ajuster en continu la tension fournie aux électrodes pour garantir une valeur fixe de la densité de courant à travers les électrodes. La densité de courant est une valeur programmable aussi bien quant à son minimum qu'à son maximum. Le logiciel permet de piloter de manière indépendante, plusieurs électrodes.

Par exemple :
- en cas d'élévation brutale de la température, le logiciel arrête le protocole d'administration du courant et émet une alerte pouvant être lumineuse ou sonore ...
- en cas de chute de la résistivité en deçà d'une valeur plancher prédéfinie et programmable, le logiciel arrête le protocole d'administration du courant et émet une alerte pouvant être lumineuse ou sonore ...
- En cas d'élévation de la résistivité au-delà d'une valeur maximale prédéfinie et programmable, le logiciel arrête le protocole d'administration du courant et émet une alerte pouvant être lumineuse ou sonore ...

Afin de garantir la sécurité, le dispositif comprend des éléments discrets comme des composants électroniques passifs pour effectuer en parallèle la mesure du courant et corriger si nécessaire la tension de sortie de l'élévateur de tension pour limiter le courant selon une consigne prédéfinie ayant une valeur minimale et une valeur maximale. On entend par composants électroniques passifs, des composants ne présentant pas de programmation possible ou encore des composants fonctionnant d'eux-mêmes tels des diodes, des capacités ou encore des résistances.

Selon une possibilité préférée, le module électronique comprend un dispositif d'écrêtage interdisant toute valeur de courant supérieure à 1mA. Le dispositif d'écrêtage est préférentiellement un dispositif indépendant du logiciel embarqué décrit ci-dessus. Le dispositif d'écrêtage comprend par exemple une diode Zener.

Selon un mode de réalisation préféré, le boitier 8 est séparable du support 1. Préférentiellement, le boitier 8 est réutilisable. Le support 1 est lui avantageusement un consommable qui va être utilisé une seule fois ou quelque fois mais dans une mesure plus faible que le boitier 8. Le support 1 est jetable. Ceci est particulièrement intéressant d'un point de vue écologique et économique car le support 1 comprend les électrodes 4. Or, le déposant a pu constater que les électrodes des dispositifs d'électrostimulation et/ou iontophorèse subissaient de forte oxydation et donc un endommagement au cours du temps. Cette disposition permet de jeter le support 1 après utilisation sans le boitier 8, ou d'administrer plusieurs substances actives ou bien faire alternativement de l'électrostimulation et de l'iontophorèse uniquement en changeant de support 1.

Le dispositif selon l'invention permet d'avoir un dispositif qui puisse à la fois être à usage unique ou du moins utilisé pour un seul patient tout en réutilisant les éléments électroniques coûteux.

Selon l'invention, le support 1 pouvant également se nommer timbre est une nappe souple. La souplesse est telle que deux points opposés du pourtour peuvent être accolés, sans détérioration du support 1. Cette souplesse est avantageusement maintenue lorsque le boitier 8 est connecté et fixé sur le support 1. Préférentiellement, le support 1 présente une épaisseur de 40 à 60 µm. La souplesse du support 1 permet au dispositif de se conformer à la zone d'application du corps où il est destiné à être appliqué.

Le dispositif selon l'invention est utilisable sur l'ensemble des zones du corps humain facilement et sans gêne ou désagrément pour l'utilisateur.

Le support 1 présente une surface supérieure à celle du boitier 8. Plus précisément, la surface de la première face 2a du support 1 est plus grande que la surface du boitier 8 en regard du support 1. Avantageusement, le ratio des surfaces première face 2a / boitier 8 est au moins égale à 1/5. Cette différence de taille permet notamment de maintenir une souplesse du support pour une bonne application sur toute la zone à traiter.

Préférentiellement, le boitier 8 à une surface en regard du support 1 de l'ordre de 1 à 6 cm². Préférentiellement la première face 2a du support 1 est de l'ordre de 8 à 50 cm².

A titre d'exemple, le support 1 a une épaisseur de 50µm et des dimensions de 41mm de largueur pour 142 mm de longueur.

La surface du support 1 est avantageusement adaptable à la surface sur laquelle il est destiné à être appliqué. A titre d'exemple illustré aux figures 5 et 6, le support 1 comprend une portion centrale destinée à coopérer avec l'encoche 10 du boitier 8, de part et d'autre de la portion centrale s'étend au moins une portion élargie portant au moins deux électrodes 4. Avantageusement, le support 1 est sécable pour s'adapter à la surface sur laquelle il est destiné à être appliqué. Dans le mode de réalisation illustré en figure 6 dans lequel le support 1 comprend deux portions élargies s'étendant de part et d'autres de la portion centrale, le support 1 peut être sectionné entre deux portions élargies pour réduire la surface d'application du support 1. La souplesse du support 1 permet avantageusement cette coupure.

Le dispositif selon l'invention peut être utilisé dans des applications cosmétiques ou thérapeutiques. Par exemple, si le dispositif est utilisé pour l'électrostimulation, le courant pénètre dans la peau pour, soit stimuler les fibroblastes de manière à augmenter leur taux de production de collagène et d'élastine dont l'effet est une amélioration de l'élasticité de la peau, soit traiter une cicatrisation. Si le dispositif est utilisé pour l'iontophorèse, il destiné à faire pénétrer des substances actives dans le derme, telles que par exemple la vitamine A ou rétinol ayant un effet dépigmentant, la lidocaïne ayant un effet anesthésique local, l'acide hyaluronique ayant des propriété régénératrices et cicatrisantes, l'acide rétinoïque pour le traitement de l'acné, la vitamine C ayant un effet antioxydant, un chélateur d'ions tel que l'acide β-alanine di-acétique pour le traitement des érythèmes, l'acide glycolique améliorant la texture de la peau, le phosphate sodique de dexaméthasone ayant un effet anti-inflammatoire ou tout autre type d'actif ionisé ou présenté sous forme d'émulsions anioniques ou cationiques afin de pouvoir être véhiculé par le courant.

Le module électronique 3 est configuré pour délivrer un courant d'intensité contrôlée. A titre préféré, l'intensité I est inférieure ou égale à 1mA.

Préférentiellement, le support 1 comprend un matériau plastique. A titre d'exemple, le support 1 est en polyimide (PI), polyéthylène téréphtalate (PET), polyéther éther cétone (PEEK), polyéthylène naphthalate (PEN).

Selon un autre mode de réalisation, le support 1 comprend une trame textile par exemple du nylon.

Selon un autre mode de réalisation, le support 1 est réalisé en papier ou papier synthétique.

Le matériau du support 1 est préférentiellement fin. Le matériau du support 1 est préférentiellement résistant à des températures élevées pour des éventuels recuits de l'encre conductrice.

Le support 1 est appliqué seul pour de l'électrostimulation ou avec une couche intercalaire pour l'iontophorèse.

Selon un mode de réalisation, le support 1 comprend de la biocellulose préférentiellement imbibée par exemple de substances actives pour l'iontophorèse.

Selon un autre mode de réalisation, le dispositif comprend un gel contenant de substances actives pour l'iontophorèse. Le gel peut être appliqué directement sur la peau puis le support 1 est appliqué par-dessus ou bien le gel est appliqué sur le support 1 puis l'ensemble est ensuite appliqué sur la peau. Le gel peut former les moyens de fixations du support 1 sur la peau notamment en présentant des propriétés d'adhésion.

Selon un autre mode de réalisation, le dispositif comprend un polymère adhésif contenant de substances actives pour l'iontophorèse.

A titre d'exemple non limitatif, les moyens de fixation type adhésif et/ou la couche intercalaire contenant des substances actives mesurent de 25 à 60 µm d'épaisseur.

Le module électronique 3 comprend selon des possibilités, des capteurs des paramètres physiologiques tels que la température, le pH, la pression, la résistivité et/ou des témoins lumineux tels que des LEDs (diodes électroluminescentes)

Selon une possibilité avantageuse, le dispositif comprend une pluralité d'anodes et une pluralité de cathodes alternées avec les anodes. Dans cette configuration, le courant total se divise suivant plusieurs circuits anode-cathode de manière à ce que la densité du courant circulant d'une anode à une cathode soit préférentiellement inférieure à 1mA/cm2. En outre, afin d'empêcher la formation de lignes de champ externes, c'est-à-dire à l'extérieur du dispositif, les deux électrodes se trouvant aux deux extrémités sont de même polarité, c'est à dire que ce sont deux anodes ou deux cathodes.

### REFERENCES

- 1: Support
- 2a.: Première face
- 2b.: Deuxième face
- 3: Module électronique
- 4: Electrodes
- 5: Moyens de connexion
- 6: Circuit électrique
- 7: Moyens de détachement partiel
- 8: Boitier
- 9: Connecteur à ressort
- 10: Encoche
- 11a.: Première face
- 11b.: Deuxième face
- 12: Clip
- 13: clip
- 14: Recharge
- 15: Relief
- 16: Connecteur électrique fixe
- 17: Fond
- 18: Bouton de commande central
- 19: Signal lumineux
- 20: Capteurs

## Revendications

1. Dispositif pour l'électrostimulation et/ou l'iontophorèse comprenant un boitier (8) comprenant un module électronique (3) et un support (1) amovible présentant une première face (2a) destinée à être placée au regard de la peau d'un utilisateur et portant des électrodes (4) configurées pour être au contact de la peau et une deuxième face (2b) opposée à la première, le support est destiné à être connecté électriquement au boitier (8), **caractérisé en ce que** le boitier (8) comprend une première face (11a) et une deuxième face (11b) destinée à être appliqué sur la peau d'un utilisateur et opposée à la première face et des moyens de connexion électriques comprenant au moins un connecteur à ressort (9) agencé sur ladite première face (11a) du boitier (8) et destiné à assurer la connexion électrique avec le support (1) et dans lequel le support (1) comprend sur sa première face (2a) un circuit électrique (6) reliant les électrodes (4) et **en ce que** le support (1) comprend sur sa première face (2a) un élément conducteur destiné à coopérer avec le au moins un connecteur à ressort (9).

2. Dispositif selon la revendication précédente dans lequel le support (1) comprend une nappe souple, et dans lequel la nappe souple présente une surface supérieure à la surface du boitier (8).

3. Dispositif selon l'une quelconque des revendications précédentes dans lequel le connecteur à ressort (9) est un plot de connexion à ressort.

4. Dispositif selon l'une quelconque des revendications précédentes dans lequel l'élément conducteur est une portion du circuit électrique (6).

5. Dispositif selon l'une quelconque des revendications précédentes comprenant un film de matériau isolant électriquement destiné à isoler électriquement ledit circuit électrique (6) de la peau sur laquelle le dispositif est destiné à être appliqué.

6. Dispositif selon l'une quelconque des revendications précédentes dans lequel le circuit électrique (6) et les électrodes (4) sont formés par une encre conductrice imprimée ou sérigraphiée sur la première face (2a) du support (1).

7. Dispositif selon l'une quelconque des revendications précédentes dans lequel le boitier (8) comprend sur sa première face (11a) une encoche (10) destinée à recevoir au moins partiellement le support (1) et dans laquelle est agencé le au moins un connecteur à ressort (9).

8. Dispositif selon la revendication précédente comprenant une recharge (14) amovible configurée pour coopérer avec l'encoche (10) du boitier (8) de sorte à maintenir le support (1) agencé entre le boitier (8) et la recharge (14), le support (1) étant au contact du au moins un connecteur à ressort (9) du boitier (8).

9. Dispositif selon la revendication précédente dans laquelle la recharge (14) comprend au moins un élément actif cosmétique ou thérapeutique destiné à diffuser sur le support (1).

10. Dispositif selon l'une quelconque des deux revendications précédentes dans lequel la recharge (14) et l'encoche (10) sont destinées à coopérer par des fixations rapides.

11. Dispositif selon l'une quelconque des revendications précédentes dans lequel le boitier (8) comprend sur une deuxième face (11b) opposée à la première face (11a) des connecteurs électriques fixes (16) destinés à être en connexion électrique avec le support (1), ou en connexion électrique direct avec la peau de l'utilisateur.

12. Dispositif selon l'une quelconque des revendications précédentes dans lequel le support (1) comprend au moins un élément actif cosmétique ou thérapeutique disposé sur sa première face (2a).

13. Dispositif selon la revendication précédente dans lequel le support (1) comprend une trame en biocellulose imbibé sur la première face (2a) du support (1) d'au moins un élément actif, ou une trame textile étant recouverte sur la première face (2a) du support (1) d'un hydrogel contenant au moins un élément actif.

14. Dispositif selon l'une quelconque des revendications précédentes dans lequel le support (1) comprend un adhésif sur sa première face (2a) de sorte à maintenir le dispositif appliqué sur la peau, avantageusement dans lequel l'adhésif est une matrice polymère formulée contenant au moins un élément actif.

15. Procédé d'assemblage d'un dispositif pour l'électrostimulation et/ou l'iontophorèse selon la revendication 8 **caractérisé en ce qu'**il comprend l'application du support (1) dans l'encoche (10) assurant la mise en contact de l'élément conducteur du support (1) et du connecteur à ressort (9) et le placement de la recharge (14) dans l'encoche (10) maintenant en position le support dans l'encoche (10).

## Patentansprüche

1. Vorrichtung zur Elektrostimulation und/oder lontophorese, umfassend ein Gehäuse (8), das ein Elektronikmodul (3) umfasst, und einen abnehmbaren Träger (1), der eine erste Seite (2a) aufweist, die dazu bestimmt ist, der Haut eines Benutzers zugewandt platziert zu werden, und die Elektroden (4) trägt, die dafür konfiguriert sind, sich mit der Haut in Kontakt zu befinden, und eine der ersten gegenüberliegende zweite Seite (2b), der Träger dazu bestimmt ist, elektrisch mit dem Gehäuse (8) verbunden zu werden, **dadurch gekennzeichnet, dass** das Gehäuse (8) eine erste Seite (11a) und eine zweite Seite (11b) umfasst, die der ersten Seite gegenüberliegt und dazu bestimmt ist, auf die Haut eines Benutzers aufgelegt zu werden, und elektrische Verbindungsmittel, die mindestens einen Federverbinder (9) umfassen, der auf der ersten Seite (11a) des Gehäuses (8) angeordnet und dazu bestimmt ist, die elektrische Verbindung mit dem Träger (1) sicherzustellen, und wobei der Träger (1) auf seiner ersten Seite (2a) eine elektrische Schaltung (6) umfasst, die die Elektroden (4) anschließt, und dadurch, dass der Träger (1) auf seiner ersten Seite (2a) ein leitendes Element umfasst, das dazu bestimmt ist, mit dem mindestens einen Federverbinder (9) zusammenzuwirken.

2. Vorrichtung nach dem vorstehenden Anspruch, wobei der Träger (1) eine flexible Lage aufweist, und wobei die flexible Lage eine Oberfläche aufweist, die höher ist als die Oberfläche des Gehäuses (8).

3. Vorrichtung nach einem der vorstehenden Ansprüche, wobei der Federverbinder (9) ein Federverbindungspad ist.

4. Vorrichtung nach einem der vorstehenden Ansprüche, wobei das leitende Element ein Teilstück der elektrischen Schaltung (6) ist.

5. Vorrichtung nach einem der vorstehenden Ansprüche, das einen Film aus elektrisch isolierendem Material umfasst, der dazu bestimmt ist, die elektrische Schaltung (6) elektrisch von der Haut zu isolieren, auf die die Vorrichtung aufgelegt werden soll.

6. Vorrichtung nach einem der vorstehenden Ansprüche, wobei die elektrische Schaltung (6) und die Elektroden (4) von einer leitenden Tinte gebildet werden, die auf die erste Seite (2a) des Trägers (1) gedruckt oder siebgedruckt ist.

7. Vorrichtung nach einem der vorstehenden Ansprüche, wobei das Gehäuse (8) auf seiner ersten Seite (11a) eine Vertiefung (10) umfasst, die dazu bestimmt ist, den Träger (1) mindestens teilweise aufzunehmen, und in der der mindestens eine Federverbinder (9) angeordnet ist.

8. Vorrichtung nach dem vorstehenden Anspruch, die eine herausnehmbare Nachfüllpatrone (14) umfasst, die dafür konfiguriert ist, so mit der Vertiefung (10) des Gehäuses (8) zusammenzuwirken, dass der Träger (1) zwischen dem Gehäuse (8) und der Nachfüllpatrone (14) angeordnet gehalten wird, wobei sich der Träger (1) mit dem mindestens einen Federverbinder (9) des Gehäuses (8) in Kontakt befindet.

9. Vorrichtung nach dem vorstehenden Anspruch, wobei die Nachfüllpatrone (14) mindestens ein kosmetisches oder therapeutisches Wirkstoffelement umfasst, das dazu bestimmt ist, sich auf dem Träger (1) zu verteilen.

10. Vorrichtung nach einem der zwei vorstehenden Ansprüche, wobei die Nachfüllpatrone (14) und die Vertiefung (10) dazu bestimmt sind, über Schnellbefestigungen zusammenzuwirken.

11. Vorrichtung nach einem der vorstehenden Ansprüche, wobei das Gehäuse (8) auf einer der ersten Seite (11a) gegenüberliegenden zweiten Seite (11b) feste elektrische Verbinder (16) umfasst, die dazu bestimmt sind, sich mit dem Träger (1) in elektrischer Verbindung, oder direkt mit der Haut des Benutzers in elektrischer Verbindung zu befinden.

12. Vorrichtung nach einem der vorstehenden Ansprüche, wobei der Träger (1) mindestens ein kosmetisches oder therapeutisches Wirkstoffelement umfasst, das auf seiner ersten Seite (2a) disponiert ist.

13. Vorrichtung nach dem vorstehenden Anspruch, wobei der Träger (1) ein Gewebe aus Biozellulose umfasst, das auf der ersten Seite (2a) des Trägers (1) mit mindestens einem Wirkstoffelement getränkt ist, oder ein Textilgewebe, das auf der ersten Seite (2a) de Trägers (1) mit einem Hydrogel beschichtet ist, das mindestens ein Wirkstoffelement umfasst.

14. Vorrichtung nach einem der vorstehenden Ansprüche, wobei der Träger (1) auf seiner ersten Seite (2a) einen Kleber umfasst, um die Vorrichtung auf der Haut aufgelegt zu halten, wobei der Kleber vorteilhafterweise eine Polymermatrix ist, die so formuliert ist, dass sie mindestens ein Wirkstoffelement umfasst.

15. Verfahren zum Zusammenbauen einer Vorrichtung zur Elektrostimulation und/oder lontophorese nach Anspruch 8, **dadurch gekennzeichnet, dass** es das Auflegen des Trägers (1) in der Vertiefung (10), das die Kontaktierung des leitenden Elements des Träges (1) und des Federverbinders (9) sicherstellt, und das Platzieren der Nachfüllpatrone (14) in der Vertiefung (10) umfasst, die den Träger in der Vertiefung (10) in Position hält.

## Claims

1. Electrostimulation and/or iontophoresis device comprising a casing (8) comprising an electronic module (3) and a removable support (1) having a first face (2a) intended to be placed facing the skin of a user and carrying electrodes (4) configured to be in contact with the skin and a second face (2b) opposite the first face, the support is intended to be connected electrically to the casing (8), **characterised in that** the casing (8) comprises a first face (11a) and a second face (11b) intended to be applied to the skin of a user and opposite the first face and electrical connection means comprising at least one spring connector (9) arranged on said first face (11a) of the casing (8) and intended to ensure the electrical connection with the support (1) and wherein the support (1) comprises on the first face (2a) thereof, an electrical circuit (6) connecting the electrodes (4) and **in that** the support (1) comprises on the first face (2a) thereof, a conductive element intended to cooperate with the at least one spring connector (9).

2. Device according to the preceding claim, wherein the support (1) comprises a flexible layer, and wherein the flexible layer has a surface area greater than the surface area of the casing (8).

3. Device according to any one of the preceding claims, wherein the spring connector (9) is a spring connection pad.

4. Device according to any one of the preceding claims, wherein the conductive element is a portion of the electrical circuit (6).

5. Device according to any one of the preceding claims comprising an electrically insulating material film intended to electrically insulate said electrical circuit (6) from the skin on which the device is intended to be applied.

6. Device according to any one of the preceding claims, wherein the electrical circuit (6) and the electrodes (4) are formed by a printed or screen-printed conductive ink on the first face (2a) of the support (1).

7. Device according to any one of the preceding claims, wherein the casing (8) comprises on the first face (11a) thereof, a groove (10) intended to receive at least partially the support (1) and wherein is arranged the at least one spring connector (9).

8. Device according to the preceding claim comprising a removable filler (14) configured to cooperate with the groove (10) of the casing (8) so as to maintain the support (1) arranged between the casing (8) and the filler (14), the support (1) being in contact with at least one spring connector (9) of the casing (8).

9. Device according to the preceding claim, wherein the filler (14) comprises at least one cosmetic or therapeutic active element intended to diffuse on the support (1).

10. Device according to any one of the two preceding claims, wherein the filler (14) and the groove (10) are intended to cooperate by quick securing.

11. Device according to any one of the preceding claims, wherein the casing (8) comprises on a second face (11b) opposite the first face (11a) of the secured electrical connectors (16) intended to be electrically connected with the support (1), or directly electrically connected with the skin of the user.

12. Device according to any one of the preceding claims, wherein the support (1) comprises at least one cosmetic or therapeutic active element arranged on the first face (2a) thereof.

13. Device according to the preceding claim, wherein the support (1) comprises a soaked biocellulose grid on the first face (2a) of the support (1) of at least one active element, or a textile grid being covered on the first face (2a) of the support (1) of a hydrogel containing at least one active element.

14. Device according to any one of the preceding claims, wherein the support (1) comprises an adhesive on the first face (2a) thereof so as to maintain the device applied on the skin, advantageously, wherein the adhesive is a formulated polymer matrix containing at least one active element.

15. Method for assembling a electrostimulation and/or iontophoresis device according to claim 8, **characterised in that** it comprises the application of the support (1) in the groove (10) ensuring the putting into contact of the conductive element of the support (1) and of the spring connector (9) and the placement of the filler (14) in the groove (10) maintaining in position the support in the groove (10).
